# EUROPEAN PATENT SPECIFICATION

(11) **EP 2 194 992 B1**
(45) Date of publication and mention of the grant of the patent: **21.05.2014**
(21) Application number: 08806341.7
(22) Date of filing: 18.09.2008
(51) Int. Cl.: A61K 35/36, A61K 8/98, A61Q 7/00, C12N 5/071, A61K 8/02

(54) **EPIDERMAL STIMULATION TO ENHANCE HAIR FOLLICLE FORMATION**
EPIDERMALE STIMULATION ZUR VERSTÄRKUNG DER HAARFOLLIKELBILDUNG
STIMULATION ÉPIDERMIQUE POUR AMÉLIORER LA FORMATION DE FOLLICULES PILEUX

(30) Priority: 21.09.2007 US 994691 P
(43) Date of publication of application: 16.06.2010
(73) Proprietor: Aderans Research Institute, Inc., Marietta, GA 30067 (US)
(72) Inventor: KEMP, Paul, Manchester M23 9QR (GB); TEUMER, Jeffrey, Woburn MA 01801 (US); COOLEY, Jerry, Charlotte NC 28210 (US)
(74) Representative: HOFFMANN EITLE
(86) International application number: PCT/GB2008/003186
(87) International publication number: WO 2009/037474

(56) References cited:
- WO-A-98/47471
- WO-A-03/068248
- WARREN R ET AL: "STIMULATION OF HUMAN SCALP PAPILLA CELLS BY EPITHELIAL CELLS" ARCHIVES OF DERMATOLOGICAL RESEARCH, SPRINGER, INTERNATIONAL, BERLIN, DE, vol. 286, no. 1, 1 January 1994 (1994-01-01), pages 1-05, XP000616615 ISSN: 0340-3696
- COOLEY JERRY: "Follicular cell implantation: an update on "hair follicle cloning"." FACIAL PLASTIC SURGERY CLINICS OF NORTH AMERICA MAY 2004, vol. 12, no. 2, May 2004 (2004-05), pages 219-224, XP009109903 ISSN: 1064-7406

## Description

This invention relates to hair follicle formation following implantation of follicular cells such as dermal papilla (DP cells).

Hair regeneration through the implantation of cultured follicular cells, such as DP cells, has been demonstrated in rodent models. In addition, there is proof of principle that these cells can be implanted into humans in order to induce hair formation to treat hair loss.

Other types of follicular cells which could be implanted include dermal sheath (DS) cells and/or outer root sheath (ORS) cells.

The hair shafts themselves are formed from specialised epidermal cells under instruction from the DP cells.

It is suggested that this occurs by one or a combination oftwo processes. Hair shafts may be formed by DP cells rejuvenating small vellus hairs by contributing to the epidermal cell signalling process resulting in thicker hair shafts. So-called "follicular neogenesis" may occur where the DP cells recruit inter-follicular epidermal keratinocytes and trans-differentiate them into follicular epidermal cells which in turn develop into a hair follicle and produce a hair shaft.

The process therefore requires active follicular (e.g. DP/DS/ORS) cells producing as yet unidentified signals and competent epidermal cells that can respond appropriately to the signal(s).

Demonstrations of hair induction in rodent models, while successful, are inefficient and do not directly instruct methods to implant cells in humans. This is due to inherent differences in the structure of skin between the species. Methods to implant DP cells into human subjects that will efficiently result in high levels of hair formation are required.

Stimulation of keratinocyte proliferation through "tape stripping" has been demonstrated. This method uses adhesive tape to remove the outer, cornified layers of keratinocytes and results in the stimulation of cell division in the lower layer of keratinocytes (see Potten et al., 2000, Cell Proliferation 33(4): 231-246).

Also, "tape stripping" has been used for removal of intact hairs in the anagen phase from one or more donor regions in such a way that the bulb is still attached to the hair removed (GHO'ST HOLDING B.V.: WO98/47471).

It has been mentioned in the literature that wound healing concomitant with DP cell implantation will stimulate hair formation in mice (McElwee et. al., 2003, J. Invest. Dermatology. 121: 121267-121275). However, the method used for wounding (cutting with a needle and tunnelling under the epidermis) is not appropriate for use in human subjects. Similarly, wounding by full thickness excision wounds and scalpel cuts is also not suitable.

The present invention is a method to improve the efficiency of hair formation from follicular cell implantation.

According to the invention there is provided a method for enhancing hair follicle formation by follicular cells when implanted in an outer skin surface of a recipient (for example, in close proximity to epithelial cells which generate hair), the method comprising removing keratinocytes from the outer skin surface of the recipient by epidermal stimulation (ES) and, prior to or after the ES, implanting the follicular cells under the outer skin surface of the recipient.

In another aspect, there is provided a method for enhancement of hair follicle formation by follicular cells when implanted in an outer skin surface of a recipient (for example, in close proximity to epithelial cells which generate hair), wherein ES is achieved by removal of keratinocytes from the outer skin surface, and the cells are implanted under the outer skin surface either before or after ES has been carried out.

The method uses ES to stimulate keratinocytes in the subject's skin in order to make them more receptive to the hair inductive signals originating from the implanted follicular cells. Surprisingly, the method results in (i) enhanced hair follicle formation, and (ii) improved hair growth.

The enhancement of hair follicle formation according to the invention may result in more and/or thicker hairs being formed following implantation of hair follicle cells, compared with implantation without ES.

Without being bound by theory, the inventors consider that actively proliferating keratinocytes are more receptive to hair induction by implanted dermal papilla (DP) cells and/or dermal sheath (DS) cells and/or outer root sheath (ORS) cells. Proliferating keratinocytes are more mobile (capable of movement and migration in the epidermis) than resting keratinocytes. Proliferation and migration of keratinocytes are hallmarks of hair formation.

According to the invention, ES is achieved by removal of some keratinocytes from the skin surface. The term "removal of keratinocytes" does not require the complete removal of a keratinocyte layer.

ES can be performed immediately before the cell implantation, or it can be performed at a short time after implantation (say, within a few days to a week after).

Preferably the ES is carried out on the scalp of a subject who is to receive (or has recently received) implanted cells. Other skin areas may be treated by ES to enhance hair follicle formation and improve hair growth.

One method for ES is by tape stripping. This method uses an adhesive tape (for example, Gorilla tape^{RTM} or Sellotape^{RTM}) to remove keratinocytes from the skin surface. In particular, tape stripping may use an adhesive tape or patch to remove the stratum corneum (the outer skin layer formed of dead keratinocytes), resulting in ES.

An alternative to tape stripping is abrasion (sometimes referred to as "dermabrasion"), in which an abrasive material (e.g. sandpaper or similar, such as surgical tip cleaners like the Universal Electro-Surgical Tip Cleaner [Universal Hospital Supplies, London, UJ, Product Code: UN58200]) is used to abrade the skin. Dermabrasion removes the outer keratinocyte layers (e.g. including the stratum corneum and/or other keratinocyte layers beneath the stratum corneum such as (in order) the stratum lucidum, the stratum granulosum, the stratum spinosum and/or the stratum basale). This achieves the same result as with tape stripping, namely the stimulation of keratinocyte proliferation and migration as they compensate to replace the lost layers.

Further alternative means for achieving ES are chemical peeling and minor skin surface damage by wounding (e.g. scratching, scraping or shaving).

The invention may be performed for a strictly cosmetic purpose.

The method of the invention may be performed by implanting DP cells.

Other types of follicular cells which could be implanted include DS cells and/or ORS cells.

In addition to implanting DP cells and/or DS cells and/or ORS cells, other follicular cells (e.g. additional keratinocytes) could be added to the cell mix in order to improve the efficiency of hair formation.

In a preferred embodiment, follicular cells (e.g. DP cells, DS cells, and/or ORS cells) are implanted into humans using methods taught in, e.g., US Patent Appl. Nos 2003/0161815 and 2005/0147652. For example, the follicular cells may be implanted using a syringe (for example, a Hamilton syringe) fitted with a repeating dispenser (for example, a PB600-1 repeating dispenser, used for example with a Hamilton syringe such as a Gastight® LT syringe).

Specific embodiments of the present invention will now be described without limitation and with reference to the accompanying figure:

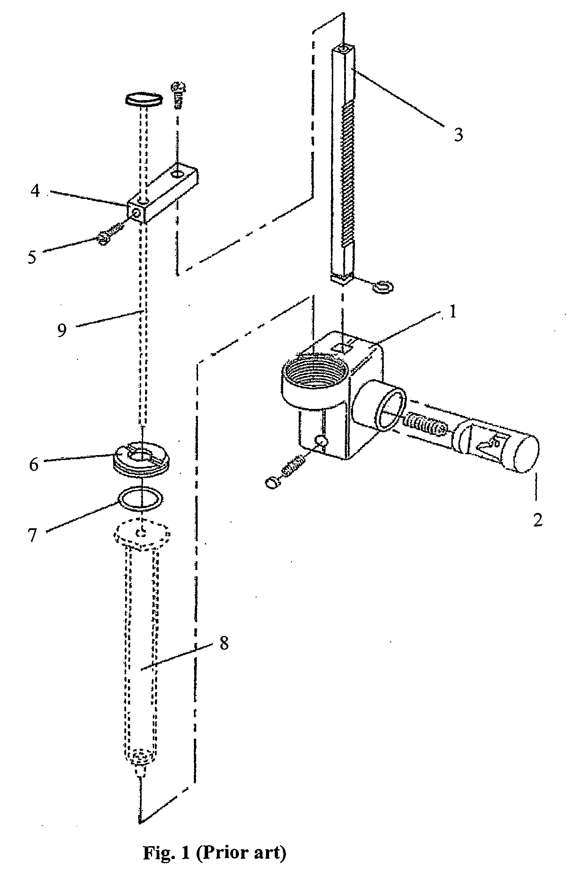

Fig. 1 is a perspective view of a prior art repeating dispenser for use with a delivery device in the method of the invention.

### Example 1. Enhancement of hair follicle formation.

First, hair is trimmed or shaved from the area to be stripped. The area is then cleaned to remove dirt and oils, as well as to remove clipped hairs. Tape stripping is performed by placing the adhesive side of tape (preferably a tape with an adhesive that strongly adheres to skin, such as Gorilla tape^{RTM} or Sellotape^{RTM}) onto the skin. The tape is removed by sharp pulling. This process is repeated until the tape appears clean and does not appear to have removed any material from the skin. Usually the skin reddens and appears irritated.

Tape stripping can be performed immediately before the cell implantation, or it can be performed at a short time after implantation (say, within a few days to a week after).

### Example 2. Comparison of tape stripping and dermabrasion methods for enhancement of hair follicle formation.

### Methods and Materials

### Materials required

In addition to the materials mentioned in the detailed method description below, a Hamilton syringe with controlled delivery modification (a "controlled delivery device", for example as exemplified in US Patent Appl. Nos 2003/0161815 and 2005/0147652 and as shown in Fig. 1), needles (as determined by the user) and a screwdriver were required.

The controlled delivery modification depicted in Fig. 1 is a Hamilton PB600-1 repeating dispenser (Hamilton Company, US), which comprises a dispenser assembly (1) with a plunger (2) and an index rod (3). The index rod is attached to a plunger arm (4) which is secured by means of a plunger arm screw (5) to a plunger (shown as outline 9) of a Hamilton syringe. A flange screw (6) secures a barrel (shown as outline 8) of the syringe in the dispenser assembly. The syringe and dispenser assembly connection is sealed by an O-ring (7).

### Patients

The patient group consisted of volunteers exhibiting male pattern baldness. Eight weeks prior to the start of the experiment, a scalp biopsy was taken from each patient for the purpose of culturing autologous DP cells. After the injection protocol (see below), hair growth was monitored and recorded at 1, 6, 12, 24, 36 and 48 weeks.

### Setting up an ICX-TRC Delivery Device

Vials containing an ICX-TRC cell suspension in polypropylene tubes, sealed in an outer plastic bag, were opened and together with appropriate media placed in a rack and kept in the fridge until use. ICX-TRC cell suspension was a 200 µl suspension of autologous human DP cells in Hypothermosol^{RTM}. Each vial contained about 8x10⁶ DP cells, prepared from a master solution of 4x10⁷ DP cells/ml cultured from each patient.

Prior to attachment of a needle to the delivery device, the injection device was primed by filling and dispensing the syringe with media twice. If the ICX-TRC cell suspension had settled to the bottom of the vial, the vial was shaken down or "flicked" to resuspend the cells. With reference to Fig. 1, the plunger tip on the injection device was checked to ensure it was positioned slightly above the 50 µl mark when the index rod (3) was fully extended. If the plunger was incorrectly positioned, the plunger arm screw (5) was loosened with a sterile screwdriver, the plunger repositioned and the screw retightened with the screwdriver.

Still without a needle attached, the syringe was carefully filled with ICX-TRC cell suspension. An appropriate needle was attached to the filled syringe and, with the needle tip facing upwards, the dispense button was pressed until an amount of ICX-TRC just began to appear at the needle tip. The needle hub has a volume of approximately 30-35 µl.

With a needle attached, the syringe was filled so that the syringe barrel contained 45-50 µl of ICX-TRC. The ICX-TRC was used within 15 minutes of opening the vial.

When the needle became blunt, a fresh needle was applied to the device and the device re-primed before continuing.

### Dispensing and handling procedures for ICX-TRC

### (i) Preparation of Scalp for ICX-TRC Injection

The target treatment area was prepared before the ICX-TRC package was opened. Before surgery the scalp was carefully washed with an antiseptic scrub taking care not to disturb the biopsy wound, and semi-dried with a clean towel. If necessary, oral diazepam was given during the procedure for sedation.

A tattoo (small black dot) was used to identify the 1cm² demarcated injection site. The area selected for each subject and the position and size of area was then documented on a diagram in a case report form (CRF), and photographs taken (see below).

Using a gentian violet pen, a 1cm² circle (with the tattoo dot in the centre) was dotted and split into 2 halves, as described below.

The selected area was then anaesthetised by injecting 0.5-1% Lignocaine with 1 in 200,000 adrenaline into the scalp surrounding the area and a prick test was performed to ensure the area was numb.

Where applicable, the selected area was subjected to epidermal stimulation as described below immediately prior to injection of the ICX-TRC cells.

At the time of use, the number of tubes present, colour of medium, packaging etc., was recorded in the CRF.

If there was any question about the sterile condition of the ICX-TRC, or if in removing the material from the package or in its handling the sterility was compromised, it was not used.

### (ii) Delivery/dispensing of ICX-TRC

On confirmation that it was acceptable to open the ICX-TRC packaging, the following instructions were applied to remove the ICX-TRC from its package.

With sterile, gloved hands, the sealed outer plastic bag was opened, the polypropylene tubes removed and transferred into the sterile field. Prior to placing the tube into a tube stand, the tube was shaken vigorously to move as much ICX-TRC to the bottom of the tube as possible.

The lid of the tube was gently screwed open, and the injector device primed and loaded as described above.

The ICX-TRC was then injected into the selected treatment area.

The following injection regimen was used (ensuring all injections were no deeper than 2mm).

In the right half of the demarcated injection area, a single step interfollicular injection procedure comprising 50 injections with a 27G-4mm needle was performed.

In the left half of the demarcated injection area, one of the following three injection procedures was performed:
(1) 50 x 1µl injections with two steps of 20G or 21G needle punch followed by injection using 27G-4mm needle;
(2) 50 x 1µl intrafollicular injections into existing follicles with 27G-4mm needle; and
(3) 1 x 50µl intradermal injection with 27G-1/2" needle.

The injection procedures are described in more detail below.

### Infection Procedures

### For single step interfollicular:

Using the Hamilton syringe and a 27G-4mm needle, the needle was pushed into the scalp, carefully avoiding any follicles. The dispenser button was clicked to deliver the cells, then the needle was slowly withdrawn to avoid creating excessive suction. Pressing down on the scalp with the hand while holding the injector when injecting was avoided, as this could compress the skin and squeeze the cells out of previous injections. Wiping or dabbing was also avoided. Working from the bottom upwards was preferred, so that bleeding/oozing did not run into new injections. It was preferred to wait before wiping, as this allowed cells to become fixed in the needle track by clotting, etc. The device was periodically checked to ensure that the plunger was advancing. The screw was tightened if needed. This check was performed at the beginning and periodically (for example, after every refill).

### For two step interfollicular:

A sharp needle (20G or 21 G) was used to make the holes, inserting only to the top of the bevel to control depth, which was about 2mm. Punches were made first, then the above single step interfollicular technique was used. A dull needle from the above single step interfollicular technique was used as this made it is easier to insert into the hole.

### For single step intrafollicular (existing follicle):

This was the same as the single step interfollicular technique except the needle was inserted directly into an existing miniature follicle. This was most easily accomplished with a needle that was dull. In some patients, the density of miniaturised follicles was not sufficient to allow 50 injections in the target area. As many injections as possible (up to 50) were made, and the number of injections recorded.

### For single step intradermal:

The needle was inserted at a point on the midline of the injection area and at the edge of the circle. The needle was pushed intradermally until the tip was at a point halfway on the radius at 90° with the midline. The needle was inserted approx. 0.5cm as shallow as possible underneath the skin such that its outline was visible under the skin. When the plunger was depressed, there was resistance - the skin blanched, and a bleb appeared. The plunger (not the dispenser button) was depressed completely, injecting the entire 50 µl contents of the syringe in a single injection.

All injections were made to the top of the head, within the selected region. The number, depth and angle of injections made were noted and documented on the CRF. In addition, the time taken to perform the injections and number of tubes used was documented. The quality of the skin was also documented including the presence of itching, oedema and erythema.

Immediately following treatment, it was recorded whether the subject experienced pain during the procedure, and the level of bleeding that was experienced.

Any unused ICX-TRC was suitably disposed of.

The following agents were acceptable for direct contact with ICX-TRC: Ciprofloxacin (drops), Mupirocin (topical) and Neomycin Sulfate (topical).

### Stimulation using adhesive tape:

Adhesive tape (Gorilla tape^{RTM} or Sellotape^{RTM}) was placed on the skin and removed by sharp pulling. The process was repeated until the tape was clean and no longer appeared to have removed any additional material from the skin.

### Stimulation using dermabrasion:

The skin was abraded to remove the outer keratinocyte layer by using a Universal Electro-Surgical Tip Cleaner (Universal Hospital Supplies, London, UJ, Product Code: UN58200). Abrasion was continued until pin-point bleeding was observed.

### Results:

In the majority of patients tested, epidermal stimulation was found to enhance the number of hairs observed in patients following injection of the ICX-TRC hair follicle cells, compared with non-epidermal stimulation controls. Representative patients showing enhanced hair formation are shown in Table 1 for epidermal stimulation by tape stripping (patients 1-3) or dermabrasion (patients 4-7), recorded at 1 week, 6 weeks, 12 weeks, 24 weeks, 36 weeks and 48 weeks post injection (where available).

**Table 1: Total number of counted hairs per half 1cm² circle**

| **Patient** | **Side** | **Technique** | **Wk1** | **Wk6** | **Wk12** | **Wk24** | **Wk36** | **Wk48** |
|---|---|---|---|---|---|---|---|---|
| 1 | RIGHT | 1 step interfollicular | 119 | 155 | 152 | - | - | - |
| | LEFT | Gorilla tape^{RTM} / 2 step interfollicular | 128 | 148 | 156 | - | - | - |
| 2 | RIGHT | 1 step interfollicular | 45 | 77 | 75 | 54 | 52 | 62 |
| | LEFT | Gorilla tape^{RTM} / 2 step interfollicular | 40 | 98 | 73 | 54 | 55 | 75 |
| 3 | RIGHT | 1 step interfollicular | 121 | 137 | 129 | NA | 144 | 123 |
| | LEFT | Sellotape^{RTM}/ 1 step intradermal | 108 | 142 | 155 | NA | 159 | 126 |
| 4 | RIGHT | 1 step interfollicular | 114 | 130 | 137 | 132 | - | - |
| | LEFT | 1 dermabrasion/ 1 step intrafollicular | 125 | 126 | 143 | 139 | - | - |
| 5 | RIGHT | 1 step interfollicular | 84 | 72 | 69 | - | - | - |
| | LEFT | dermabrasion/2 step interfollicular | 97 | 84 | 112 | - | - | - |
| 6 | RIGHT | 1 step interfollicular | 95 | 114 | 119 | - | - | - |
| | LEFT | dermabrasion/1 step intradermal | 89 | 89 | 131 | - | - | - |
| 7 | RIGHT | 1 step interfollicular | NA | 106 | 107 | - | - | - |
| | LEFT | dermabrasion/1 step intradermal | NA | 97 | 109 | - | - | - |

Furthermore, for the above patients the number of hairs of more than 30 microns in diameter was in many cases higher where epidermal stimulation was used, as shown in Table 2.

**Table 2: Number of hairs more than 30 microns in diameter per half 1cm² circle**

| **Patient** | **Side** | **Technique** | **Wk1** | **Wk6** | **Wk12** | **Wk24** | **Wk36** | **Wk48** |
|---|---|---|---|---|---|---|---|---|
| 1 | RIGHT | 1 step interfollicular | 15 | 17 | 30 | - | - | - |
| | LEFT | Gorilla tape^{RTM}/ 2 step interfollicular | 15 | 15 | 33 | - | - | - |
| 2 | RIGHT | 1 step interfollicular | 15 | 19 | 38 | 14 | 20 | 12 |
| | LEFT | Gorilla tape^{RTM} / 2 step interfollicular | 14 | 33 | 31 | 15 | 19 | 18 |
| 3 | RIGHT | 1 step interfollicular | 21 | 32 | 21 | NA | 25 | 23 |
| | LEFT | Sellotape^{RTM}/ 1 step intradermal | 14 | 23 | 21 | NA | 32 | 18 |
| 4 | RIGHT | 1 step interfollicular | 17 | 21 | 8 | 16 | - | - |
| | LEFT | dermabrasion/ 1 step intrafollicular | 16 | 16 | 9 | 16 | - | - |
| 5 | RIGHT | 1 step interfollicular | 10 | 10 | 9 | - | - | - |
| | LEFT | dermabrasion/2 step interfollicular | 5 | 7 | 12 | - | - | - |
| 6 | RIGHT | 1 step interfollicular | 4 | 5 | 5 | - | - | - |
| | LEFT | dermabrasion/1 step intradermal | 7 | 7 | 12 | - | - | - |
| 7 | RIGHT | 1 step interfollicular | NA | 28 | 27 | - | - | - |
| | LEFT | dermabrasion/1 step intradermal | NA | 16 | 10 | - | - | - |

This example shows that epidermal stimulation according to the invention enhances hair follicle formation and results in the production of more and/or thicker hairs.

### Other Embodiments

While the invention has been described in connection with specific embodiments thereof, it will be understood that it is capable of further modifications and this application is intended to cover any variations, uses, or adaptations of the invention following, in general, the principles of the invention and including such departures from the present disclosure that come within known or customary practice within the art to which the invention pertains and may be applied to the essential features herein set forth.

## Claims

1. A method for enhancing hair follicle formation by follicular cells when implanted in an outer skin surface of a recipient, the method comprising removing keratinocytes from the outer skin surface of the recipient by epidermal stimulation (ES) and, prior to or after the ES, implanting the follicular cells under the outer skin surface of the recipient at the site of epidermal stimulation, wherein the method is a cosmetic treatment.

2. The method according to claim 1, wherein the ES is carried out before implantation.

3. The method according to claim 1, wherein the ES is carried out after implantation.

4. The method according to any preceding claim, wherein the ES is achieved by tape stripping using an adhesive tape for removal of keratinocytes from the outer skin surface.

5. The method according to any preceding claim, wherein the follicular cells comprise or consist of dermal papilla (DP) cells.

6. The method according to any preceding claim, wherein the follicular cells comprise or consist of dermal sheath (DS) cells.

7. The method according to any preceding claim, wherein the follicular cells comprise or consist of outer root sheath (ORS) cells.

8. The method according to any preceding claim, wherein the follicular cells comprise or consist of a mixture of follicular cells selected from the group consisting of: dermal papilla (DP) cells, dermal sheath (DS), and outer root sheath (ORS) cells.

9. The method according to any preceding claim, wherein the follicular cells include keratinocytes.

10. The method according to any preceding claim, wherein the ES is achieved by dermabrasion.

11. The method according to any preceding claim, wherein the ES is achieved by chemical peeling.

12. The method according to any preceding claim, wherein the ES is achieved by wounding.

13. The method according to any preceding claim, wherein the follicular cells are implanted using a controlled delivery device (for example, a syringe fitted with repeating dispenser such as Hamilton syringe fitted with a PB600-1 repeating dispenser).

## Patentansprüche

1. Verfahren zur Verbesserung der Haarfollikelbildung durch Follikelzellen, wenn diese in eine äußere Hautfläche eines Empfängers implantiert werden, wobei das Verfahren die Entfernung von Keratinozyten von der äußeren Hautfläche des Empfängers durch epidermale Stimulation (ES) und, vor oder nach der ES, das Implantieren der Follikelzellen unter die äußere Hautfläche des Empfängers an der Stelle der ES umfasst, wobei das Verfahren eine kosmetische Behandlung ist.

2. Verfahren gemäß Anspruch 1, worin die ES vor der Implantation durchgeführt wird.

3. Verfahren gemäß Anspruch 1, worin die ES nach der Implantation durchgeführt wird.

4. Verfahren gemäß einem der vorhergehenden Ansprüche, worin die ES durch Abziehen eines Klebebandes zur Entfernung von Keratinozyten von der äußeren Hautfläche erreicht wird.

5. Verfahren gemäß einem der vorhergehenden Ansprüche, worin die Follikelzellen dermale Papillenzellen (DP) umfassen oder aus diesen bestehen.

6. Verfahren gemäß einem der vorhergehenden Ansprüche, worin die Follikelzellen dermale Hüllzellen (DS) umfassen oder aus diesen bestehen.

7. Verfahren gemäß einem der vorhergehenden Ansprüche, worin die Follikelzellen äußere Haarwurzelhüllzellen (ORS) umfassen oder aus diesen bestehen.

8. Verfahren gemäß einem der vorhergehenden Ansprüche, worin die Follikelzellen eine Mischung aus Follikelzellen, ausgewählt aus der Gruppe bestehend aus dermalen Papillenzellen (DP), dermalen Hüllzellen (DS) und äußeren Haarwurzelhüllzellen (ORS), umfassen oder aus dieser bestehen.

9. Verfahren gemäß einem der vorhergehenden Ansprüche, worin die Follikelzellen Keratinozyten umfassen.

10. Verfahren gemäß einem der vorhergehenden Ansprüche, worin die ES durch Dermabrasion erreicht wird.

11. Verfahren gemäß einem der vorhergehenden Ansprüche, worin die ES durch chemisches Peeling erreicht wird.

12. Verfahren gemäß einem der vorhergehenden Ansprüche, worin die ES durch Verletzung erreicht wird.

13. Verfahren gemäß einem der vorhergehenden Ansprüche, worin die Follikelzellen durch Verwendung eines kontrollierten Abgabegerätes (z.B. eine mit einer Repetierdosiervorrichtung (repeating dispenser) ausgerüstete Spritze, wie z.B. eine mit einer PB600-1 Repetierdosiervorrichtung ausgerüstete Hamilton-Spritze) implantiert werden.

## Revendications

1. Procédé pour améliorer de la formation de follicule pileux par des cellules folliculaires lorsqu'implantées dans une surface de peau extérieure d'un receveur, le procédé comprenant l'enlèvement de kératinocytes depuis la surface de peau extérieure du receveur par stimulation épidermique (ES) et, avant ou après l'ES, l'implantation des cellules folliculaire sous la surface de peau extérieure du receveur au niveau du site de stimulation épidermique, dans lequel le procédé est un traitement cosmétique.

2. Procédé selon la revendication 1, dans lequel l'ES est effectuée avant implantation.

3. Procédé selon la revendication 1, dans lequel l'ES est effectuée après implantation.

4. Procédé selon l'une quelconque des revendications précédentes, dans lequel l'ES est réalisée par décollement de bande en utilisant une bande adhésive pour enlèvement de kératinocytes depuis la surface de peau extérieure.

5. Procédé selon l'une quelconque des revendications précédentes, dans lequel les cellules folliculaires comprennent ou consistent en des cellules de papille dermique (DP).

6. Procédé selon l'une quelconque des revendications précédentes, dans lequel les cellules folliculaires comprennent ou consistent en des cellules de gaine dermique (DS).

7. Procédé selon l'une quelconque des revendications précédentes, dans lequel les cellules folliculaires comprennent ou consistent en des cellules de gaine épithéliale extérieure (ORS).

8. Procédé selon l'une quelconque des revendications précédentes, dans lequel les cellules folliculaires comprennent ou consistent en un mélange de cellules folliculaires sélectionnées à partir du groupe consistant en : des cellules de papille dermique (DP), des cellules de gaine dermique (DS), et des cellules de gaine épithéliale extérieure (ORS).

9. Procédé selon l'une quelconque des revendications précédentes, dans lequel les cellules folliculaires incluent des kératinocytes.

10. Procédé selon l'une quelconque des revendications précédentes, dans lequel l'ES est réalisée par dermabrasion.

11. Procédé selon l'une quelconque des revendications précédentes, dans lequel l'ES est réalisée par exfoliation chimique.

12. Procédé selon l'une quelconque des revendications précédentes, dans lequel l'ES est réalisée en blessant.

13. Procédé selon l'une quelconque des revendications précédentes, dans lequel les cellules folliculaires sont implantées en utilisant un dispositif de délivrance commandé (par exemple, une seringue munie d'un distributeur à répétition comme une seringue de Hamilton munie d'un distributeur à répétition PB600-1).
